# FASCICULE DE BREVET EUROPEEN

(11) **EP 0 641 436 B1**
(45) Date de publication et mention de la délivrance du brevet: **03.12.1997**
(21) Numéro de dépôt: 93910110.1
(22) Date de dépôt: 11.05.1993
(51) Int. Cl.: G01N 33/36

(54) **Procédé et installation pour l'évaluation du caractère collant de matières fibreuses végétales telles que des cotons**
Verfahren und Vorrichtung zur Beurteilung der Klebefähigkeit von faserartigen pflanzlichen Materialien wie Baumwolle
Process and facility for assessing the adhesiveness of plant fibrous materials such as cottons

(30) Priorité: 20.05.1992 FR 9206142
(43) Date de publication de la demande: 08.03.1995
(73) Titulaire: CENTRE INTERNATIONAL DE RECHERCHE AGRONOMIQUE POUR LE DEVELOPPEMENT (C.I.R.A.D.), F-34032 Montpellier (FR)
(72) Inventeur: FRYDRYCH, Richard, F-34090 Montpellier (FR)
(74) Mandataire: Lerner, François
(86) Numéro de dépôt international: FR9300457
(87) Numéro de publication internationale: WO9323752

(56) Documents cités:
- DE-A- 3 928 279
- DE-A- 4 018 847
- FR-A- 2 609 058
- US-A- 3 451 756
- PATENT ABSTRACTS OF JAPAN, vol. 12, no. 266 (P-735), 26 juillet 1988/
- Coton et Fibres Tropicales, 1986, vol. XLI, facs. 3, 211-3

## Description

L'invention a pour objet un procédé et une installation pour l'évaluation du caractère collant de matières fibreuses végétales telles que des cotons ; l'invention vise en outre l'utilisation de ce procédé et de cette installation pour la conduite dans les meilleures conditions d'opérations successives de traitement des matières fibreuses ainsi polluées.

Depuis quelques années, les matières fibreuses végétales et en particulier les cotons de diverses provenances provoquent un phénomène de collage en filature, ce qui induit de fortes pertes de productivité.

Le collage est lié principalement à des déjections d'insectes appelées "miellats" composées essentiellement de sucre et qui confèrent au coton un pouvoir collant.

Pour solutionner ce problème, plusieurs procédés ont été proposés pour déterminer et éliminer les miellats ou leur action de collage.

En particulier, la demanderesse a mis au point une machine qui permet, par examen d'un échantillon maintenu à un degré hygrométrique précis et qui est pressé entre deux feuilles d'aluminium convenablement chauffées de déterminer le potentiel de collage de l'échantillon examiné (voir Coton et Fibres Tropicales, 1986, vol. XLI, fasc. 3, 211-3).

Cette machine présente l'inconvénient que la procédure est relativement délicate à mettre en oeuvre, longue, coûteuse et qu'en outre, la détermination du degré de collage de l'échantillon reste finalement très subjective et peut varier considérablement d'un opérateur à l'autre.

L'invention a pour objet de résoudre ces difficultés en permettant une évaluation précise, rapide et économique du caractère collant de la matière fibreuse en perfectionnant notablement le procédé et la machine dont il vient d'être question.

Le procédé conforme à l'invention se caractérise par les étapes successives suivantes :
- on prépare un échantillon de la matière fibreuse de façon qu'il présente une large surface par rapport à son poids, c'est à dire de l'ordre de 100 cm² pour un poids compris entre 0,5 et 5 g,
- on applique au moyen d'un organe pressant et chauffant l'échantillon ainsi déployé sur une plaque métallique, par exemple d'aluminium ou analogue pendant quelques secondes,
- on applique ensuite une pression à froid au même échantillon sur la même plaque pendant quelques secondes,
- si nécessaire, on effectue un séchage superficiel à l'air chaud de la plaque,
- on enlève les fibres de l'échantillon adhérant à la plaque,
- et on compte le nombre des points de miellats adhérant à la plaque, lequel nombre détermine le caractère collant de l'échantillon ramené à sa surface déployée.

On partira avantageusement d'un échantillon de quelques grammes, par exemple d'un poids compris entre 2 et 5 grammes que l'on ouvrira sur une surface de quelques centaines de cm², par exemple de l'ordre de 200 cm². Lorsqu'on procède de cette façon on constate que toutes choses égales par ailleurs, le nombre des points de miellat décompté est indépendant du poids de l'échantillon qui n'a donc pas besoin d'être pesé de façon précise, tandis qu'il est aisé de l'ouvrir sur une surface donnée par exemple de 17 X 12 cm soit environ 200 cm².

Avantageusement, le chauffage s'effectue à une température comprise entre 33 et 140° C, de préférence entre 50 et 90° C.

La pression à chaud peut être maintenue pendant quelques secondes et sera de l'ordre d'au moins 40 g/cm², des résultats très satisfaisants étant obtenus avec une pression comprise entre 80 g/cm² et 500 g/cm².

Plus la température est basse, plus la pression et la durée de maintien de cette pression seront élevées.

Par exemple d'excellents résultats sont obtenus avec les paramètres suivants :
a) température : 53°C, pression : 500 g/cm², durée de maintien de la pression : 30 secondes,
b) température : 85°C, pression : 80 g/cm², durée de maintien de la pression : 5 secondes.

La pression à froid pourra être maintenue pendant 15 à 30 secondes environ, la pression étant du même ordre de grandeur que celle utilisée pour la pression à chaud.

Le procédé de l'invention permet ainsi en un laps de temps très court, inférieur à 2 minutes, de déterminer de façon précise le caractère collant d'un coton pollué par des miellats ; la connaissance quasi instantanée du pouvoir collant du coton permet alors de déterminer sans retard et d'adapter immédiatement les opérations de traitement les plus appropriées à ce coton.

En particulier, il est possible, à partir de la connaissance de ce pouvoir collant du coton, de piloter une installation de traitement de dépollution du même coton.

De la même façon, il est possible, à partir de la connaissance du pouvoir collant du coton, de déterminer quel type de filature peut éventuellement être mis en oeuvre sans inconvénient majeur.

L'invention vise en outre des installations permettant l'automatisation ou la semi-automatisation du procédé, comme il va résulter plus clairement de la description qui va suivre faite en référence aux dessins annexés dans lesquels :
la figure 1 montre schématiquement une installation pour la mise en oeuvre du procédé de l'invention selon un premier mode de réalisation,
la figure 2 montre de façon similaire à la figure 1 une variante de ce procédé,
la figure 3 est un schéma relatif à une troisième variante.

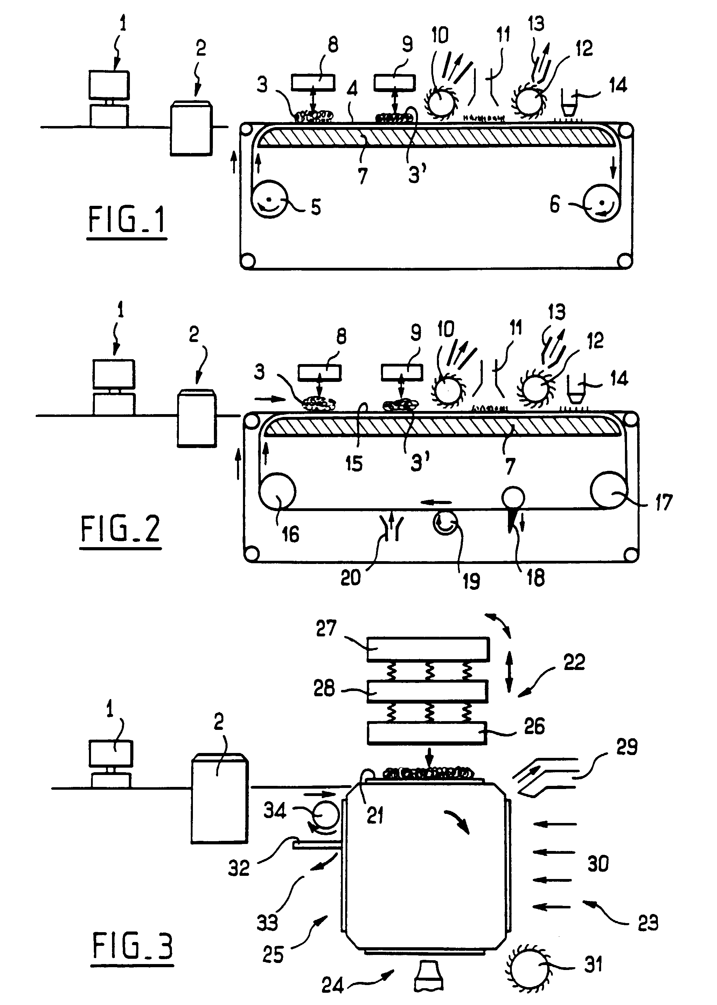

En se référant tout d'abord à la figure 1, on a repéré en 1 un système d'identification des échantillons, correspondant par exemple à des balles de coton déterminées, lesquels échantillons peuvent être repérés par un système de lecteur à codes barres par exemple.

En 2, on a représenté schématiquement un système de préparation de l'échantillon, par exemple du type à rotor permettant d'ouvrir un échantillon de coton pouvant peser entre 1 et 5 grammes, et généralement entre 2 et 3 grammes et qui une fois ouvert, va occuper une surface d'environ 17 X 12 centimètres (de l'ordre de 200 cm²).

L'échantillon est alors posé, comme indiqué en 3, sur une bande métallique, avantageusement d'aluminium 4, déroulée à partir d'un rouleau 5 approvisionneur et enroulée à la sortie de la machine sur un rouleau de reprise 6.

La feuille 4 se déroule au-dessus d'un support neutre 7 par exemple en matériau plastique ou en bois.

Un plateau chauffant 8 est chauffé à la température choisie entre 33° C et 140°C, préférentiellement entre 50° C et 90° C.

Lorsque l'échantillon 3 est en place, sous le plateau chauffant 8, celui-ci est commandé en abaissement et vient presser l'échantillon contre la feuille d'aluminium 4.

La pression est maintenue pendant une durée plus ou moins longue selon la température choisie et qui peut être de l'ordre de 5 à 30 secondes par exemple, pour des températures de l'ordre de 85° C à 53° C.

La pression est avantageusement comprise entre 40 g/cm² et 1 000 g/cm², des résultats tout à fait satisfaisants étant obtenus avec une pression comprise entre 80 g/cm² et 500 g/cm², des pressions plus élevées étant avantageusement utilisées en relation avec des températures moins élevées.

L'action conjuguée de la pression et de la chaleur exercée sur l'échantillon 3 fait s'évaporer une partie de l'humidité contenue dans le coton, créant une fine couche de vapeur sur le support d'aluminium et permettant à ce niveau le ramollissement des billes de sucre ou de miellats contenues dans le coton et qui viennent se fixer au support d'aluminium 4. A ce sujet, on notera que l'action indiquée suppose l'existence d'une certaine humidité du coton ; en l'occurrence, un degré d'humidité de l'échantillon compris entre 40 % et 85 % permet d'obtenir sans problème le résultat recherché, la mesure étant en pratique généralement conduite pour un taux d'humidité proche de 60-65 %. On notera que c'est l'effet conjugué d'une certaine épaisseur, ni trop faible, ni trop importante de l'échantillon, et d'un cheminement de vapeur à travers l'épaisseur de l'échantillon qui paraît donner à la mesure du caractère collant du coton son indépendance par rapport au poids testé de l'échantillon.

L'opération de pressage à chaud étant terminée, par exemple au bout de 5 secondes, le plateau 8 est relevé, la bande 4 est avancée d'un pas et vient se présenter sous le plateau 9 qui s'abaissera alors et qui va assurer une pression à froid de l'échantillon 3 qui s'est déplacé en 3'.

La pression à froid peut être maintenue pendant environ 15 à 30 secondes ; elle a pour objet d'assurer une meilleure fixation des points collants sur le support d'aluminium 4.

La pression du plateau froid 9 est avantageusement du même ordre de grandeur que celle exercée par le plateau chauffant 8.

A la fin de cette opération, le plateau 9 est relevé et une brosse ou un balai 10 placé après le poste 9 élimine la plupart des fibres de l'échantillon 3' lorsque la bande 4 est avancée d'un pas jusqu'au poste référencé 11 qui est un poste de séchage.

A cet endroit, l'échantillon, si nécessaire, est séché par de l'air chaud de façon à éliminer l'humidité résiduelle et à fixer correctement à la feuille d'aluminium 4 les points de sucre et de miellat qui se sont déposés sur elle. On a constaté que généralement le séchage n'est pas requis si l'on a utilisé une température de pression à chaud relativement basse, exemplairement comprise entre 50° C et 55° C et un maintien de la pression suffisamment long, exemplairement de l'ordre de 30 secondes.

L'échantillon est ensuite avancé jusqu'à une brosse 12, éventuellement doublée d'un système d'aspirateur nettoyeur 13, qui éliminent les fibres restantes adhérant à la plaque.

En 14, il ne reste plus qu'à lire, par exemple au moyen d'une caméra appropriée, le nombre de points laissés par l'échantillon sur la feuille 4.

Ce nombre de points, ramené à la surface de l'échantillon préparé, permet de déterminer de façon précise et automatique le degré ou pouvoir collant du coton ainsi traité.

Bien entendu, de nombreuses variantes peuvent être apportées au mode de réalisation décrit.

Ainsi, dans le mode de réalisation illustré à la figure 2 et dans laquelle les mêmes repères indiquent les éléments semblables se retrouvant dans ces deux réalisations et qui ne seront pas redécrits, la feuille d'aluminium 4 a été remplacée par une bande métallique continue 15, par exemple d'aluminium d'épaisseur convenable et qui circule en continu en étant entraînée sous tension entre les deux rouleaux 16, 17 dont l'un au moins est moteur.

La bande 15, à la sortie du poste 14 est débarrassée comme indiqué en 18, par exemple par un racleur de la plupart des matières qui y adhèrent, après quoi elle est convenablement nettoyée par un rouleau ou une brosse 19 éventuellement imprégné d'un solvant, puis séchée en 20, avant réutilisation au poste de départ 8.

Dans la variante illustrée à la figure 3, au lieu d'une bande métallique qui se déplace sous différents postes successifs, on trouve des plaques individuelles se déplaçant successivement sous des postes : 22 d'application du miellat sur la plaque 21 ; 23 de nettoyage/chauffage de l'échantillon pour le débarrasser des fibres ; 24 de comptage des points de miellat ; et 25 de nettoyage avant réutilisation au poste 22.

Le poste 22 peut comprendre une plaque de pressage à chaud 26 et une plaque de pressage à froid 27 qui viennent successivement en action (après retournement de 180° de l'ensemble) et qui sont séparées par un isolant 28.

Le poste de nettoyage/enlèvement des fibres, peut comprendre une zone d'aspiration/brossage 29 et une zone de séchage 30 ainsi qu'une brosse 31 pour enlever les dernières fibres.

Le poste de nettoyage 25 peut comprendre une raclette 32 avec élimination des déchets en 33 et une brosse de nettoyage/séchage 34.

Bien entendu, de nombreuses variantes peuvent être imaginées aux modes de réalisation schématiquement décrits uniquement à titre d'illustration. En particulier, on peut bien entendu travailler sur des échantillons plus ou moins importants, l'essentiel étant de respecter une proportion dans laquelle le poids de l'échantillon utilisé reste faible par rapport à sa surface, soit préférentiellement de l'ordre de 2 à 5 g pour 200 cm² dans les exemples donnés, une fourchette satisfaisante pouvant être par exemple de 0,5 à 5 g pour 100 cm2.

De la description qui précède, on comprend que le procédé et l'installation d'évaluation conformes à l'invention peuvent être entièrement automatisés et que les paramètres acquis par cette installation peuvent être utilisés pour piloter de la manière appropriée toute installation de traitement prévue en aval.

En particulier, une installation du type ci-dessus décrit peut être utilisé pour piloter une installation d'élimination des miellats notamment par dissolution/modification par vapeur d'eau chauffée, en adaptant le degré du traitement au degré de pollution du coton.

## Revendications

1. Procédé d'évaluation du caractère collant de matières fibreuses végétales telles que des cotons, caractérisé en ce que :
- on prépare un échantillon (3) de la matière fibreuse de façon qu'il présente une large surface par rapport à son poids, c'est-à-dire de l'ordre de 100 cm² pour un poids compris entre 0,5 et 5 g,
- on applique au moyen d'un organe pressant et chauffant (8) l'échantillon ainsi déployé sur une plaque métallique, par exemple d'aluminium (4, 15, 21), pendant quelques secondes,
- on applique ensuite une pression à froid au même échantillon sur la même plaque pendant quelques secondes,
- on effectue éventuellement un séchage superficiel à l'air chaud de la plaque,
- on enlève les fibres de l'échantillon qui adhèrent à la plaque,
- et on compte le nombre des points de miellats adhérant à la plaque, lequel nombre détermine le caractère collant de l'échantillon ramené à sa surface déployée.

2. Procédé selon la revendication 1 caractérisé en ce qu'on utilise en tant qu'échantillon une masse de coton de quelques grammes, avantageusement de 2 à 5 g, que l'on ouvre sur une surface de quelques centaines de cm², avantageusement de l'ordre de 200 cm².

3. Procédé selon la revendication 1 ou 2 caractérisé en ce qu'on effectue le chauffage entre 33 et 140° C, de préférence entre 50° C et 90° C.

4. Procédé selon l'une des revendications précédentes caractérisé en ce qu'on maintient la pression à chaud pendant une durée comprise entre 5 secondes et 30 secondes environ.

5. Procédé selon l'une des revendications précédentes caractérisé en ce que la pression à chaud est supérieure à 40 g/cm² et de préférence comprise entre 80 g/cm² et 500 g/cm².

6. Procédé selon les revendications 3, 4 et 5, caractérisé en ce que la valeur de la pression utilisée pour la pression à chaud et la durée du maintien de cette pression sont d'autant plus élevées dans les fourchettes indiquées que la température est plus basse.

7. Procédé selon l'une des revendications précédentes caractérisé en ce qu'on maintient la pression à froid pendant une durée d'environ 15 à 30 secondes.

8. Installation pour la mise en oeuvre du procédé selon l'une des revendications précédentes 1 à 7 caractérisée en ce qu'elle comprend :
- au moins un rouleau d'approvisionnement (5) d'une feuille continue d'aluminium (4),
- au moins un rouleau de reprise (6) de ladite feuille,
- ladite feuille (4) tendue entre les deux rouleaux (5) et (6) constituant ladite plaque sur laquelle est pressé l'échantillon.

9. Installation pour la mise en oeuvre du procédé selon l'une quelconque des revendications 1 à 7 caractérisée en ce qu'on utilise en tant que plaque sur laquelle est pressé l'échantillon une bande continue (15) nettoyée après le processus d'évaluation du collage.

10. Installation pour la mise en oeuvre du procédé selon l'une quelconque des revendications 1 à 7 caractérisée en ce qu'on utilise en tant que plaque sur laquelle est pressé l'échantillon, une plaque (21) qui est déplacée devant des postes successifs de pressage (22) à chaud et à froid de l'échantillon, de brossage-séchage (23), de comptage (24) puis de nettoyage (25).

11. Procédé de gestion d'une installation pour le traitement de matières fibreuses telles que des cotons utilisant un procédé d'évaluation du caractère collant des matières fibreuses mises en oeuvre, selon l'une quelconque des revendications 1 à 7 caractérisé en ce que l'installation de traitement est gérée en fonction des paramètres d'analyse recueillis sur des échantillons dont le caractère collant a été précédemment évalué.

## Patentansprüche

1. Verfahren zur Beurteilung der Klebeeigenschaften von pflanzenlichen Fasermaterialien so wie Baumwolle, dadurch gekennzeichnet, daß :
- man eine Probe (3) des Fasermaterials derart vorbereitet, daß sie eine große Oberfläche im Verhältnis zu ihrem Gewicht aufweist, d.h. in Bereich von 100 cm² bei einem Gewicht im Bereich zwischen 0,5 und 5 g,
- man die mittels einer Preß- und Heizeinrichtung (8) ausgebreitete Probe so auf eine Metallplatte, zum Beispiel aus Aluminium (4, 15, 21), für einige Sekungen aufträgt,
- man danach für einige Sekunden einen Kaltdruck auf dieselbe Probe auf derselben Platte ausübt,
- man gegebenenfalls eine Oberflächentrocknung mittels der Warmluft der Platte bewirkt,
- man die Fasern der Probe, die an der Platte haften, beseitigt,
- und man die Zahl der Punkte von an der Platte klebendem honigartigem Sekret zählt, die die Klebeeigenschaften der Probe, bezogen auf ihre ausgebreitete Oberfläche, bestimmt.

2. Verfahren nach Anspruch 1, dadurch gekennzeichnet, daß man als Probe eine Menge Baumwolle von einigen Gramm, vorteilhafterweise 2 bis 5 g, verwendet, die man auf einer Oberfläche von einigen 100 cm², vorteilhafterweise in der Größenordnung von 200 cm², ausbreitet.

3. Verfahren nach Anspruch 1 oder 2, dadurch gekennzeichnet, daß man die Erwärmung zwischen 33 und 140°C, bevorzugt zwischen 50°C und 90°C, bewirkt.

4. Verfahren nach einem der vorherigen Ansprüche, dadurch gekennzeichnet, daß man den Warmdruck während einer Dauer von ungefähr 5 bis 30 Sekunden ausübt.

5. Verfahren nach einem der vorherigen Ansprüche, dadurch gekennzeichnet, daß der Warmdruck größer als 40 g/cm² und vorzugsweise im Bereich zwischen 80 g/cm² und 500 g/cm² ist.

6. Verfahren nach einem der Ansprüche 3, 4 und 5, dadurch gekennzeichnet, daß die Höhe des Drucks, der für den Warmdruck angewandt wird, und die Dauer der Aufrechterhaltung dieses Drucks in den angegebenen Bereichen umso höher sind, wie die Temperatur niedriger ist.

7. Verfahren nach einem der vorherigen Ansprüche, dadurch gekennzeichnet, daß man den Kaltdruck für eine Dauer von ungefähr 15 bis 30 Sekunden aufrechterhält.

8. Einrichtung zur Durchführung des Verfahrens nach einem der vorherigen Ansprüche 1 bis 7, dadurch gekennzeichnet, daß sie umfaßt:
- mindestens eine Walze (5) zum Zuführen einer fortlaufenden Aluminiumfolienbahn (4),
- mindestens eine Walze (6) zum Wiederaufnehmen der Bahn,
- die Bahn (4), die zwischen den beiden Walzen (5) und (6) gehalten wird und die Platte darstellt, auf der auf die Probe Druck ausgeübt wird.

9. Einrichtung zur Durchführung des Verfahrens nach einem der Ansprüche 1 bis 7, dadurch gekennzeichnet, daß man als Platte, auf der auf die Probe Druck ausgeübt wird, ein fortlaufendes Band (15) verwendet, das nach dem Verfahren der Beurteilung des Klebens gereinigt wird.

10. Einrichtung zur Durchführung des Verfahrens nach einem der Ansprüche 1 bis 7, dadurch gekennzeichnet, daß man als Platte, auf der auf die Probe Druck ausgeübt wird, eine Platte (21) verwendet, die vor den aufeinanderfolgenden Stationen der Warm- und Kaltdruckausübung (22) auf die Probe, des Bürsten-Trocknens (23), des Zählens (24) und schließlich der Reinigung (25) verlagert wird.

11. Verfahren zum Steuern einer Einrichtung zur Behandlung von Fasermaterialien wie Baumwolle unter Verwendung eines Verfahrens zur Bestimmung des Klebegrads von Fasermaterialien, durchgeführt nach einem der Ansprüche 1 bis 7, dadurch gekennzeichnet, daß die Behandlungseinrichtung in Abhängigkeit von den Parametern der Analyse, die von Proben aufgenommen wurden, deren Klebeeigenschaften vorher ausgewertet wurden, gesteuert wird.

## Claims

1. Process for the evaluation of the adhesive character of vegetable fibrous materials such as cottons, characterised in that:
- a sample (3) of the fibrous material is prepared in such a manner that it has a wide surface in relation to its weight, ie. of the order of 100cm² for a weight of between 0.5 and 5g;
- the sample which is thus spread is applied to a metallic plate, for example of aluminium (4, 15, 21), by means of a pressing and heating member for several seconds;
- a cold pressure is subsequently applied to the same sample on the same plate for several seconds;
- optionally, a drying with hot air is performed at the surface of the plate;
- the fibres of the sample which adhere to the plate are removed; and
- the number of sticky points adhering to the plate is counted, this number determining the adhesive character of the sample, taken at the surface thereof which has been used.

2. Process according to Claim 1,
characterised in that a mass of cotton of a few grams, advantageously from 2 to 5g, is used as the sample, which mass of cotton is processed over a surface of some hundreds of cm², advantageously of the order of 200 cm².

3. Process according to Claim 1 or Claim 2, characterised in that the heating is performed between 33 and 140°C, and preferably between 50°C and 90°C.

4. Process according to any one of the preceding Claims, characterised in that the hot pressure is maintained for a duration of between approximately 5 seconds and 30 seconds.

5. Process according to one of the preceding Claims characterised in that the hot pressure is greater than 40 g/cm² and preferably between 80 g/m² and 500 g/cm².

6. Process according to the Claims 3, 4 and 5 characterised in that the magnitude of the pressure used for the hot pressure and the duration of maintenance of this pressure are situated higher within the ranges indicated the lower the temperature.

7. Process according to one of the preceding Claims characterised in that the cold pressure is maintained for a duration of from 15 to 30 seconds.

8. Installation for performing the process according to one of Claims 1 to 7, characterised in that it comprises:
- at least one roller (5) for supplying a continuous aluminium sheet (4);
- at least one take-up roller (6) for said sheet; and
- said sheet (4), held between the two rollers (5) and (6), forming said plate on which the sample is pressed.

9. Installation for performing the process according to any one of Claims 1 to 7,
characterised in that, there is used, as the plate onto which the sample is pressed, a continuous belt (15), which is cleaned after the process of evaluation of the adhesion.

10. Installation for performing the process according to any one of Claims 1 to 7,
characterised in that, there is used, as the plate onto which the sample is pressed, a plate (21) which is moved in front of successive hot and cold stations (22) for pressing the sample, brushing-drying (23), counting (24) and then cleaning (25).

11. Process for the management of an installation for the treatment of fibrous materials such as cottons using a process for evaluating the adhesive nature of the fibrous materials used, according to any one of Claims 1 to 7, characterised in that the treatment installation is managed as function of the analysis parameters gathered from samples the adhesive character of which has been evaluated previously.
